# EUROPÄISCHE PATENTANMELDUNG

(11) **EP 0 819 689 A1**
(43) Veröffentlichungstag der Anmeldung: **21.01.1998**
(21) Anmeldenummer: 96111557.3
(22) Anmeldetag: 18.07.1996
(51) Int. Cl.: C07D 405/06, A61K 31/34

(54) **5-substituierte (2H)Chromene**

(71) Anmelder: Byk Gulden Lomberg Chemische Fabrik GmbH, 78467 Konstanz (DE)
(72) Erfinder: Gutterer, Beate, 78476 Allensbach (DE); Flockerzi, Dieter, 78476 Allensbach (DE); Amschler, Hermann, 78315 Radolfzell (DE); Ulrich, Wolf-Rüdiger, 78464 Konstanz (DE); Bär, Thomas, 78462 Konstanz (DE); Martin, Thomas, 78464 Konstanz (DE); Hatzelmann, Armin, 78467 Konstanz (DE); Boss, Hildegard, 78464 Konstanz (DE); Beume, Rolf, 78465 Konstanz (DE); Häfner, Dietrich, 78464 Konstanz (DE); Kley, Hans-Peter, 78476 Allensbach (DE)

(57) **Zusammenfassung**

Verbindungen der Formel I, 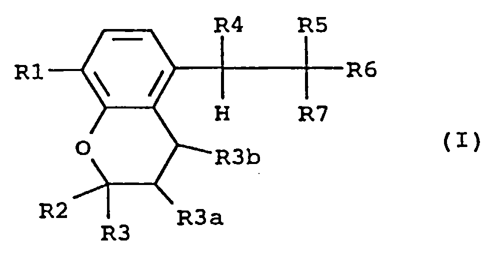 worin R1, R2, R3, R3a, R3b, R4, R5, R6, R7 und R8 die in der Beschreibung angegebenen Bedeutungen haben, sind neue wirksame Bronchialtherapeutika.

## Beschreibung

### Anwendungsgebiet der Erfindung

Die Erfindung betrifft neue 5-substituierte [2H]Chromene, die in der pharmazeutischen Industrie zur Herstellung von Medikamenten verwendet werden.

### Bekannter technischer Hintergrund

In der internationalen Patentanmeldung WO94/02465 werden Inhibitoren der c-AMP Phosphodiesterase und des TNF beschrieben. - In den internationalen Patentanmeldungen WO94/20446, WO95/35281, WO95/35283 und WO95/35284 werden in bestimmter Weise trisubstituierte Phenylderivate als Inhibitoren der Phosphodiesterase (PDE) Typ IV beschrieben. - In der internationalen Patentanmeldung WO95/20455 werden Styrylderivate, ihre Herstellung und die Verwendung als PDE-IV Inhibitoren offenbart. - In der internationalen Patentanmeldung WO95/35285 werden tetrasubstituierte Phenylderivate und ihre Verwendung als PDE-IV Inhibitoren beschrieben.

### Beschreibung der Erfindung

Es wurde nun gefunden, daß die nachfolgend näher beschriebenen neuen 5-substituierten [2H]Chromene überraschende und besonders vorteilhafte Eigenschaften besitzen.

Gegenstand der Erfindung sind somit Verbindungen der Formel I (siehe beigefügtes Formelblatt), worin
- R1: 1-4C-Alkoxy oder ganz oder überwiegend durch Fluor substituiertes 1-4C-Alkoxy bedeutet,
- R2: Wasserstoff, 1-7C-Alkyl, 3-7C-Cycloalkyl oder 3-7C-Cycloalkylmethyl bedeutet,
- R3: Wasserstoff, 1-7C-Alkyl, 3-7C-Cycloalkyl oder 3-7C-Cycloalkylmethyl bedeutet,
oder worin
- R2 und R3: gemeinsam und unter Einschluß des Kohlenstoffatoms, an das beide gebunden sind, einen 3-7C-Cycloalkylrest darstellen,
- R3a und R3b: jeweils Wasserstoff oder gemeinsam eine weitere C-C-Bindung bedeuten,
- R4: Wasserstoff oder Hydroxy bedeutet,
- R5: Wasserstoff bedeutet,
oder worin
- R4 und R5: gemeinsam eine weitere C-C-Bindung darstellen,
- R6: Wasserstoff oder Cyano bedeutet,
- R7: einen Pyridylrest bedeutet,
die Salze dieser Verbindungen sowie die N-Oxide der Pyridine und deren Salze.

1-4C-Alkoxy steht für einen Rest, der neben dem Sauerstoffatom einen geradkettigen oder verzweigten Alkylrest mit 1 bis 4 Kohlenstoffatomen enthält. Als Alkylreste mit 1 bis 4 Kohlenstoffatomen seien hierbei beispielsweise genannt der Butyl-, iso-Butyl-, sec.-Butyl-, tert.-Butyl-, Propyl-, Isopropyl-, Ethyl- und der Methylrest.

Als ganz oder überwiegend durch Fluor substituiertes 1-4C-Alkoxy seien beispielsweise der 1,2,2-Trifluorethoxy-, der 2,2,3,3,3-Pentafluorpropoxy-, der Perfluorethoxy- und insbesondere der 1,1,2,2-Tetrafluorethoxy-, der Trifluormethoxy-, der 2,2,2-Trifluorethoxy- und der Difluormethoxyrest genannt.

1-7C-Alkyl steht für geradkettige oder verzweigte Alkylreste mit 1 bis 7 Kohlenstoffatomen. Beispielsweise seien genannt der Heptyl-, Isoheptyl-(2-Methylhexyl-), Hexyl-, Isohexyl- (2-Methylpentyl-), Neohexyl- (2,2-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

3-7C-Cycloalkyl steht für den Cyclopropyl-, Cyclobutyl-, Cyclopentyl-, Cyclohexyl- und Cycloheptylrest. Bevorzugt seien die 3-5C-Cycloalkylreste Cyclopropyl, Cyclobutyl und Cyclopentyl genannt.

3-7C-Cycloalkylmethyl steht für einen Methylrest, der durch einen der vorstehend genannten 3-7C-Cycloalkylreste substituiert ist. Bevorzugt seien die 3-5C-Cycloalkylmethylreste Cyclopropylmethyl, Cyclobutylmethyl und Cyclopentylmethyl genannt.

Der Pyridylrest R7 kann über die 2-, 3- oder 4-Position an den Rest der Verbindung der Formel I angeknüpft sein, wobei eine Anbindung Ober die 3- oder 4-Position bevorzugt ist.

Wenn R2 und R3 gemeinsam und unter Einschluß des Kohlenstoffatoms, an das beide gebunden sind, einen 3-7C-Cycloalkylrest darstellen, so liegt eine Spiroverbindung vor.

Hervorzuhebende Verbindungen der Formel I sind solche, worin
- R1: 1-2C-Alkoxy bedeutet,
- R2: Wasserstoff, 1-4C-Alkyl oder 3-5C-Cycloalkyl bedeutet,
- R3: Wasserstoff, 1-4C-Alkyl oder 3-5C-Cycloalkyl bedeutet,
oder worin
- R2 und R3: gemeinsam und unter Einschluß des Kohlenstoffatoms, an das beide gebunden sind, einen 3-7C-Cycloalkylrest darstellen,
- R3a und R3b: jeweils Wasserstoff oder gemeinsam eine weitere C-C-Bindung bedeuten,
- R4: Wasserstoff oder Hydroxy bedeutet,
- R5: Wasserstoff bedeutet,
oder worin
- R4 und R5: gemeinsam eine weitere C-C-Bindung darstellen,
- R6: Wasserstoff oder Cyano bedeutet,
- R7: einen Pyridylrest bedeutet,
sowie die Salze dieser Verbindungen.

Besonders hervorzuhebende Verbindungen der Formel I sind solche, worin
- R1: 1-2C-Alkoxy bedeutet,
- R2: 1-4C-Alkyl oder 3-5C-Cycloalkyl bedeutet,
- R3: Wasserstoff oder 1-4C-Alkyl bedeutet,
oder worin
- R2 und R3: gemeinsam und unter Einschluß des Kohlenstoffatoms, an das beide gebunden sind, einen Cyclopropyl-, Cyclobutyl- oder Cyclopentylring darstellen, und
- R3a und R3b: jeweils Wasserstoff oder gemeinsam eine weitere C-C-Bindung bedeuten,
- R4: Wasserstoff oder Hydroxy bedeutet,
- R5: Wasserstoff bedeutet,
oder worin
- R4 und R5: gemeinsam eine weitere C-C-Bindung darstellen,
- R6: Wasserstoff oder Cyano bedeutet,
- R7: einen Pyridylrest bedeutet,
sowie die Salze dieser Verbindungen.

Bevorzugte Verbindungen der Formel I sind solche, worin
- R1: Methoxy bedeutet,
- R2: 1-4C-Alkyl bedeutet und
- R3: 1-4C-Alkyl bedeutet,
oder worin
- R2 und R3: gemeinsam und unter Einschluß des Kohlenstoffatoms, an das beide gebunden sind, einen Cyclopropyl-, Cyclobutyl- oder Cyclopentylring darstellen,
- R3a und R3b: jeweils Wasserstoff oder gemeinsam eine weitere C-C-Bindung bedeuten,
- R4: Wasserstoff bedeutet,
- R5: Wasserstoff bedeutet,
oder worin
- R4 und R5: gemeinsam eine weitere C-C-Bindung darstellen,
- R6: Wasserstoff oder Cyano bedeutet,
- R7: einen Pyridylrest bedeutet,
oder
- R1: Methoxy bedeutet,
- R2: 1-4C-Alkyl bedeutet und
- R3: 1-4C-Alkyl bedeutet,
oder worin
- R2 und R3: gemeinsam und unter Einschluß des Kohlenstoffatoms, an das beide gebunden sind, einen Cyclopropyl-, Cyclobutyl- oder Cyclopentylring darstellen,
- R3a und R3b: jeweils Wasserstoff oder gemeinsam eine weitere C-C-Bindung bedeuten,
- R4: Hydroxy bedeutet,
- R5: Wasserstoff bedeutet,
- R6: Wasserstoff bedeutet,
- R7: einen Pyridylrest bedeutet,
sowie die Salze dieser Verbindungen.

Als Salze kommen für Verbindungen der Formel I insbesondere alle Säureadditionssalze in Betracht. Besonders erwähnt seien die pharmakologisch verträglichen Salze der in der Galenik üblicherweise verwendeten anorganischen und organischen Säuren. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Verbindungen der Formel I, worin R4 und R5 gemeinsam eine weitere C-C-Bindung darstellen, können als cis- oder trans-Isomere vorliegen. Je nach Bedeutung der Substituenten R2, R3, R4, R5 und R6 können die Verbindungen der Formel I auch ein oder mehrere Chiralitätszentren besitzen. Die Erfindung umfaßt daher sowohl die reinen Enantiomeren und Diastereomeren (einschließlich der cis/trans-Isomeren) als auch deren Gemische in jedem Mischungsverhältnis, einschließlich der Racemate. Die Enantiomeren können in an sich bekannter Weise (beispielsweise durch Herstellung und Trennung entsprechender diastereoisomerer Verbindungen) separiert werden (siehe z.B. WO92/08716).

Weiterer Gegenstand der Erfindung ist ein Verfahren zur Herstellung der Verbindungen der Formel I und ihrer Salze, sowie der N-Oxide der Pyridine und deren Salze. Das Verfahren ist dadurch gekennzeichnet, daß man
a) zur Herstellung von Verbindungen der Formel I, worin R1, R2 und R3 die oben genannten Bedeutungen besitzen, R3a und R3b gemeinsam eine C-C-Bindung bedeuten, R4 Hydroxy bedeutet, R5 und R6 Wasserstoff bedeuten und R7 die oben genannten Bedeutungen besitzt, entsprechende Verbindungen der Formel II (siehe beigefügtes Formelblatt), worin R1, R2 und R3 die oben genannten Bedeutungen besitzen mit entsprechenden Verbindungen der Formel M-C(R5)(R6)(R7), worin R5 und R6 Wasserstoff bedeuten und R7 die oben genannten Bedeutungen besitzt und M ein geeignetes Metallatom darstellt, umsetzt oder daß man
b) zur Herstellung von Verbindungen der Formel I, worin R1, R2, R3, R6 und R7 die oben genannten Bedeutungen besitzen und worin R3a, R3b, R4 und R5 Wasserstoff bedeuten, entsprechende Verbindungen der Formel I, worin R1, R2, R3, R6 und R7 die oben genannten Bedeutungen besitzen und worin R3a gemeinsam mit R3b und/oder R4 gemeinsam mit R5 eine weitere C-C-Bindung bedeutet hydriert, oder daß man
c) zur Herstellung von Verbindungen der Formel I, worin R1, R2, R3, R3a, R3b, R6 und R7 die oben genannten Bedeutungen besitzen und R4 und R5 gemeinsam eine C-C-Bindung bedeuten, entsprechende Verbindungen der Formel I, worin R1, R2, R3, R3a, R3b, R6 und R7 die oben genannten Bedeutungen besitzen, R4 Hydroxy und R5 Wasserstoff bedeutet, dehydratisiert, oder daß man
d) zur Herstellung von Verbindungen der Formel I, worin R1, R2, R3 und R7 die oben genannten Bedeutungen besitzen, R3a und R3b sowie R4 und R5 jeweils gemeinsam eine C-C-Bindung bedeuten und R6 Cyano bedeutet, entsprechende Verbindungen der Formel II, worin R1, R2 und R3 die oben genannten Bedeutungen haben mit entsprechenden Verbindungen der Formel NC-CH₂-R7, worin R7 die oben genannten Bedeutungen hat, in Gegenwart einer Base umsetzt,
und daß man gewünschtenfalls anschließend nach a), b), c) oder d) erhaltene Verbindungen der Formel I in ihre Salze oder in die N-Oxide der Pyridine und gewünschtenfalls anschließend in die Salze überführt, oder daß man gewünschtenfalls anschließend erhaltene Salze der Verbindungen der Formel I in die freien Verbindungen überführt.

Die Umsetzung der Verbindungen der Formel II mit metallorganischen Verbindungen der Formel M-C(R5)(R6)(R7) nach Verfahrensvariante a) erfolgt auf dem Fachmann bekannte Weise in einem geeigneten Lösungsmittel wie einem Ether, beispielsweise einem cyclischen Ether wie Tetrahydrofuran und vorzugsweise bei niedrigen Temperaturen, insbesondere bei Temperaturen zwischen -70°C und Raumtemperatur. Verbindungen der Formel M-C(R5)(R6)(R7), worin R5 und R6 Wasserstoff bedeuten und R7 die oben genannten Bedeutungen hat und worin M ein geeignetes Metallatom, beispielsweise Lithium bedeutet, sind entweder bekannt oder können auf bekannte Weise hergestellt werden, beispielsweise durch Umsetzung entsprechender Verbindungen der Formel H-C(R5)(R6)(R7) mit einer Alkyllithiumverbindung (wie z.B. Butyllithium) unter bekannten Reaktionsbedingungen.

Die Hydrierung analog Verfahrensvariante b) erfolgt unter üblichen Hydrierungsbedingungen, Vorzugsweise durch Verwendung von Wasserstoff in Gegenwart eines geeigneten Katalysators wie Palladium auf Aktivkohle und in einem geeigneten Lösungsmittel, beispielsweise einem Alkohol wie Ethanol oder einem Ether wie Tetrahydrofuran und beispielsweise bei Raumtemperatur.

Die Dehydratisierung von Verbindungen der Formel I, worin R4 Hydroxy und R5 Wasserstoff bedeutet, erfolgt analog bekannter Dehydratisierungsmethoden, insbesondere in Gegenwart einer Säure, wie beispielsweise Toluolsulfonsäure in einem inerten organischen Lösungsmittel, wie beispielsweise Toluol und vorzugsweise bei erhöhter Temperatur, beispielsweise bei der Siedetemperatur des verwendeten Lösungsmittels. Gewünschtenfalls kann die Hydroxylgruppe R4 auch vor der Eliminierung geeignet derivatisiert werden, insbesondere durch Überführung in einen Ester, beispielsweise einen Sulfonsäureester oder einen Phosphorsäureester, wobei die Eliminierung dann vorzugsweise in Gegenwart einer Base, beispielsweise einer Stickstoffbase wie Pyridin oder Triethylamin erfolgt.

Die Umsetzung analog Variante d) erfolgt auf dem Fachmann bekannte Weise in einem geeigneten Lösungsmittel, vorzugsweise einem polaren, protischen Lösungsmittel, beispielsweise in einem Alkohol wie Methanol oder Ethanol, in Wasser oder in einem Wasser-Alkohol-Gemisch und in Gegenwart einer geeigneten Base, vorzugsweise eines Hydroxids wie Natrium- oder Kaliumhydroxid, beispielsweise bei Raumtemperatur. Verbindungen der Formel NC-CH₂-R7 sind bekannt oder können auf bekannte Weise hergestellt werden. Für die oben genannte Umsetzung können sie als solche oder auch in Form ihrer Salze eingesetzt werden.

Beispielsweise erfolgen die Umsetzungen nach den Verfahrensvarianten a), b) c) oder d) so wie in den nachfolgenden Beispielen beschrieben oder analog den in der internationalen Patentanmeldungen WO 94/20446 oder WO 94/20455 beschriebenen Verfahren.

Die N-Oxidation erfolgt auf eine dem Fachmann ebenfalls vertraute Weise, z.B. mit Hilfe von m-Chlorperoxibenzoesäure in Dichlormethan bei Raumtemperatur. Welche Reaktionsbedingungen für die Durchführung des Verfahrens im einzelnen erforderlich sind, ist dem Fachmann aufgrund seines Fachwissens geläufig.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, einem Ether wie Diethylether oder Tetrahydrofuran, oder einem niedermolekularen aliphatischen Alkohol (Ethanol, Isopropanol), das die gewünschte Säure enthält, oder dem die gewünschte Säure anschliessend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die Verbindungen der Formel II, worin R1, R2 und R3 die oben angegebenen Bedeutungen haben, sind durch eine Claisen-Umlagerung der entsprechend substituierten Verbindungen der Formel III (siehe beigefügtes Formelblatt) zugänglich (z.B. so, wie in J. Med. Chem. **1983,** 26 (11), 1585 oder Chem. Pharm. Bull. **1992,** 40(5), 1148-1153 und dort zitierter Literatur beschrieben).

Die Verbindungen der Formel III sind entweder bekannt oder können auf dem Fachmann bekannte Weise aus bekannten Ausgangsverbindungen, wie z.B. in Tetrahedron Lett. **1994,** 35, 6405-6408 oder in den nachfolgenden Beispielen beschrieben, hergestellt werden.

Die nachfolgenden Beispiele dienen der näheren Erläuterung der Erfindung ohne sie einzuschränken. Ebenso können weitere Verbindungen der Formel I, deren Herstellung nicht explizit beschrieben ist, in analoger oder in einer dem Fachmann an sich vertrauten Weise unter Anwendung üblicher Verfahrenstechniken hergestellt werden.

In den Beispielen steht Schmp. für Schmelzpunkt, Sdp. für Siedepunkt, h für Stunde(n), Min. für Minute(n), RT für Raumtemperatur, SF für Summenformel, MG für Molgewicht, Ber. für Berechnet, Gef. für Gefunden, abs. für absolut, d.Th. für der Theorie. Die in den Beispielen genannten Verbindungen und ihre Salze sowie die N-Oxide der Pyridine und deren Salze sind bevorzugter Gegenstand der Erfindung.

### Beispiele

### Endprodukte

### 1. (±)-Spiro{5-[1-Hydroxy-2-(pyrid-4-yl)ethyl]-8-methoxy-[2H]chromen-2,1'-Cyclopentan}

1,0 g 4-Picolin werden in 30 ml abs. Tetrahydrofuran gelöst und bei -70°C mit einer Lösung aus 6,65 ml 15 %igem Butyllithium in Hexan und 10 ml abs. Tetrahydrofuran versetzt. Nach 15 Min. tropft man 2,5 g Spiro{8-Methoxy-[2H]chromen-5-carbaldehyd-2,1'-Cyclopentan} in 30 ml abs. Tetrahydrofuran zu und erwärmt über Nacht unter Rühren auf RT. Die Lösung wird im Vakuum eingeengt, der Rückstand mit Essigsäureethylester/Wasser extrahiert, die organische Phase mit Natriumsulfat getrocknet, das Lösungsmittel im Vakuum eingeengt und der Rückstand an neutralem Kieselgel mit einem Gemisch aus Essigsäureethylester/Petrolether/Triethylamin im Verhältnis 8/2/0,5 chromatographiert. Nach Eindampfen der Produktfraktionen erhält man 1,4 g (40,7 % d.Th.) der Titelverbindung mit Schmp. 140-141°C.
SF: C₂₁ H₂₃ N O₃; MG: 337,42

| | | | | |
|---|---|---|---|---|
| Elementaranalyse: | Ber.: | C 74,75 | H 6,87 | N 4,15 |
| | Gef.: | C 74,87 | H 6,80 | N 3,95 |

### 2. (E)-Spiro{5-[2-(pyrid-4-yl)ethenyl]-8-methoxy-[2H]chromen-2,1'-Cyclopentan}

3,1 g Spiro{5-[1-Hydroxy-2-(pyrid-4-yl)ethyl]-8-methoxy-[2H]chromen-2,1'-Cyclopentan} werden in 50 ml Toluol suspendiert, mit 3,6 g Toluolsulfonsäure Monohydrat versetzt und 4 h unter Rückfluß erwärmt. Das Reaktionsgemisch wird in Essigsäureethylester aufgenommen, mit gesättigter Natriumhydrogencarbonatlösung extrahiert, die organische Phase mit Natriumsulfat getrocknet und im Vakuum eingeengt. Nach Umkristallisation aus Ethanol erhält man 2,65 g (90,2 % d.Th.) der Titelverbindung mit Schmp. 111-112°C.
SF: C₂₁ H₂₁ N O₂; MG: 319,41

| | | | | |
|---|---|---|---|---|
| Elementaranalyse: | Ber.: | C 78,79 | H 6,63 | N 4,39 |
| | Gef.: | C 78,67 | H 6,42 | N 4,29 |

### 3. Spiro{5-[2-(pyrid-4-yl)ethyl]-8-methoxychroman-2.1'-Cyclopentan}

1,2 g Spiro{5-[2-(pyrid-4-yl)ethenyl]-8-methoxy-[2H]chromen-2,1'-Cyclopentan} werden in 15 ml abs. Tetrahydrofuran gelöst, mit 20 mg Palladium/Kohle 10 %ig versetzt und 10 h bei Normaldruck hydriert. Das Reaktionsgemisch wird filtriert, das Lösungsmittel im Vakuum eingeengt und der Rückstand an neutralem Kieselgel mit einem Gemisch aus Petrolether/Essigsäureethylester/Triethylamin im Verhältnis 4/4/1 chromatographiert. Nach Eindampfen der Produktfraktionen erhält man 900 mg (74,2 % d.Th.) der Titelverbindung mit Schmp. 84,5-85,5°C.
SF: C₂₁ H₂₅ N O₂; MG: 323,44

| | | | | |
|---|---|---|---|---|
| Elementaranalyse: | Ber.: | C 77,98 | H 7,79 | N 4,33 |
| | Gef.: | C 77,91 | H 7,88 | N 4,52 |

### 4. (Z)-Spiro{5-[2-Cyano-2-(pyrid-4-yl)ethenyl]-8-methoxy-[2H]chromen-2.1'-Cyclopentan}

3,4 g 4-Pyridylacetonitril Hydrochlorid und 1,88 g Natriumhydroxid werden in 17 ml Wasser suspendiert und bei RT mit einer Lösung von 5,0 g Spiro{8-Methoxy-[2H]chromen-5-carbaldehyd-2,1'-Cyclopentan} in 30 ml Ethanol versetzt. Der entstehende Niederschlag wird abgesaugt und mit Ethanol/Wasser im Verhältnis 1/1 nachgewaschen. Nach Umkristallisation aus Ethanol erhält man 4,6 g (65,1 % d.Th.) der Titelverbindung mit Schmp. 159-160°C.
SF: C₂₂ H₂₀ N₂ O₂; MG: 344,42

| | | | | |
|---|---|---|---|---|
| Elementaranalyse: | Ber.: | C 76,72 | H 5,82 | N 8,13 |
| | Gef.: | C 76,69 | H 5,77 | N 7,97 |

Ausgehend von 8-Methoxy-2,2-dimethyl-[2H]chromen-5-carbaldehyd erhält man durch Umsetzung mit 4-Pyridylacetonitril Hydrochlorid entsprechend der Arbeitsweise nach Beispiel 4:

### 5. (Z)-5-[2-Cyano-2-(pyrid-4-yl)ethenyl]-8-methoxy-2,2-dimethyl-[2H]chromen

Schmp.: 138°C, Ausbeute 84,0 % d.Th.
SF: C₂₀ H₁₈ N₂ O₂; MG: 318,4

| | | | | |
|---|---|---|---|---|
| Elementaranalyse: | Ber.: | C 75,45 | H 5,70 | N 8,80 |
| | Gef.: | C 75,58 | H 5,76 | N 8,82 |

Ausgehend von 8-Methoxy-2,2-dimethyl-[2H]chromen-5-carbaldehyd erhält man durch Umsetzung mit 3-Pyridylacetonitril entsprechend der Arbeitsweise nach Beispiel 4:

### 6. (Z)-5-[2-Cyano-2-(pyrid-3-yl )ethenyl]-8-methoxy-2,2-dimethyl-[2H]chromen

Schmp.: 225°C (Hydrochlorid), Ausbeute 46,0 % d.Th.
SF: C₂₀ H₁₈ N₂ O₂ X HCl; MG: 354,8

| | | | | | |
|---|---|---|---|---|---|
| Elementaranalyse: | Ber.: | C 67,70 | H 5,40 | Cl 9,99 | N 7,90 |
| | Gef.: | C 67,83 | H 5,53 | Cl 10,03 | N 7,78 |

Ausgehend von 8-Methoxy-2,2-dimethyl-[2H]chromen-5-carbaldehyd erhält man durch Umsetzung mit 2-Pyridylacetonitril entsprechend der Arbeitsweise nach Beispiel 4:

### 7. (Z)-5-[2-Cyano-2-(pyrid-2-yl)ethenyl]-8-methoxy-2,2-dimethyl-[2H]chromen

Schmp.: 99°C, Ausbeute 78,0 % d.Th.
SF: C₂₀ H₁₈ N₂ O₂, MG: 318,4

| | | | | |
|---|---|---|---|---|
| Elementaranalyse: | Ber.: | C 75,45 | H 5,70 | N 8,80 |
| | Gef.: | C 75,40 | H 5,67 | N 8,73 |

### 8. (±)-Spiro{5-[2-Cyano-2-(pyrid-4-yl)ethyl]-8-methoxychroman}

1,0 g (Z)-Spiro{5-[2-Cyano-2-(pyrid-4-yl)ethenyl]-8-methoxy-[2H]chromen-2,1'-Cyclopentan} werden in 15 ml abs. Tetrahydrofuran gelöst, mit 100 mg Palladium/Kohle 10 %ig versetzt und 70 h bei Normaldruck hydriert. Nach Filtration und Entfernen des Lösungsmittels im Vakuum wird der Rückstand an neutralem Kieselgel mit einem Gemisch aus Essigsäureethylester/Petrolether im Verhältnis 2/1 chromatographiert. Die Produktfraktionen werden im Vakuum eingedampft, der Rückstand in Diethylether aufgenommen und mit Diethylether/HCl versetzt. Der Niederschlag wird abgesaugt und getrocknet. Man erhält 580 mg (52,0 % d.Th.) der Titelverbindung mit Schmp. 181,5°C (Hydrochlorid).
SF: C₂₂ H₂₄ N₂ O₂ x HCl, MG: 384,91

Ausgehend von (Z)-5-[2-Cyano-2-(pyrid-4-yl)ethenyl]-8-methoxy-2,2-dimethyl-[2H]chromen erhält man durch Hydrierung entsprechend der Arbeitsweise nach Beispiel 8:

### 9. (±)-5-[2-Cyano-2-(pyrid-4-yl)ethyl]-8-methoxy-2,2-dimethylchroman

Schmp.: 146°C, Ausbeute 25,0 % d.Th.
SF: C₂₀ H₂₂ N₂ O₂, MG: 322,4

| | | | | |
|---|---|---|---|---|
| Elementaranalyse: | Ber.: | C 74,50 | H 6,88 | N 8,69 |
| | Gef.: | C 74,44 | H 6,87 | N 8,57 |

Ausgehend von (Z)-5-[2-Cyano-2-(pyrid-2-yl)ethenyl]-8-methoxy-2,2-dimethyl-[2H]chromen erhält man durch Hydrierung entsprechend der Arbeitsweise nach Beispiel 8:

### 10. (±)-5-[2-Cyano-2-(pyrid-2-yl)ethyl]-8-methoxy-2,2-dimethylchroman

Schmp.: 214°C (Hydrochlorid), Ausbeute 38,0 % d.Th.
SF: C₂₀ H₂₂ N₂ O₂ x HCl, MG: 322,4

| | | | | | |
|---|---|---|---|---|---|
| Elementaranalyse: | Ber.: | C 66,94 | H 6,46 | Cl 9,88 | N 7,81 |
| | Gef.: | C 66,82 | H 6,41 | Cl 9,74 | N 7,64 |

### Ausgangsverbindungen

### A. Spiro{8-Methoxy-[2H]chromen-5-carbaldehyd-2,1'-cylopentan}

13,0 g 3-(1-Ethinylcyclopentyloxy)-4-methoxybenzaldehyd werden in 100 ml Diethylanilin gelöst und 8 h bei 120°C gerührt. Das Lösungsmittel wird im Vakuum entfernt, der Rückstand in 200 ml Diethylether aufgenommen und dreimal mit 80 ml 2 M Salzsäure extrahiert. Nach Trocknen der organischen Phase mit Natriumsulfat und Entfernen des Lösungsmittels im Vakuum erhält man 12,2 g (93,8 % d.Th.) der Titelverbindung als erstarrendes Öl mit Schmp. 71,5-73°C.

In gleicher Weise erhält man ausgehend von 3-(1,1-Dimethylprop-2-inyloxy)-4-methoxybenzaldehyd entsprechend der Arbeitsweise nach Beispiel A:

### B. 2,2-Dimethyl-8-methoxy-[2H]chromen-5-carbaldehyd

Schmp.: 74,5-75°C, Ausbeute 78,9 % d.Th.

### C. 3-(1-Ethinylcyclopentyloxy)-4-methoxybenzaldehyd

### Lösung 1:

40,0 g Ethinylcyclopentanol werden unter Stickstoffbegasung in 180 ml trockenem Acetonitril gelöst, mit Eis/Kochsalz auf -5°C gekühlt, 70,0 ml 1,8-Diazabicyclo(5,4,0)undec-7-en (DBU) zugegeben, die Mischung 10 Min. bei -5°C gerührt und anschließend 50,8 ml Trifluoressigsäureanhydrid so zugetropft, daß die Temperatur der Lösung unter 0°C gehalten wird. Nach beendeter Zugabe wird die Lösung für weitere 30 Min. bei -5° bis -2°C gerührt.

### Lösung 2:

60,7 g Isovanillin werden unter Stickstoffbegasung in 180 ml trockenem Acetonitril gelöst, mit Eis/Kochsalz auf -5°C gekühlt, 0,1 g Kupfer-(I)-chlorid und 76,3 ml DBU zugegeben und die Mischung bei -5°C weitere 30 Min. gerührt.

Lösung 1 wird nun unter Rühren bei -5°C während 40 Min. in die Lösung 2 eingetropft und die Mischung bei 0°C 5 h gerührt. Anschließend wird das Gemisch im Vakuum eingedampft, der Rückstand in 500 ml Wasser aufgenommen und dreimal mit je 300 ml Toluol extrahiert. Die vereinigten Toluol-Extrakte werden nacheinander mit dreimal 200 ml 1 N Salzsäure, zweimal 200 ml 1 N Natronlauge, 100 ml gesättigter Natriumbicarbonatlösung und schließlich mit 100 ml gesättigter Kochsalzlösung gewaschen und über geglühtem Magnesiumsulfat getrocknet. Nach Entfernen des Lösungsmittels im Vakuum erhält man 33,7 g (38 % d.Th.) der Titelverbindung als Öl.

In gleicher Weise erhält man aus Isovanillin und 2-Methyl-3-butin-2-ol entsprechend der Arbeitsweise nach Beispiel C:

### D. 3-(1,1-Dimethylprop-2-inyloxy)-4-methoxybenzaldehyd

Öl, Ausbeute 46,0 % d.Th.

### Gewerbliche Anwendbarkeit

Die erfindungsgemäßen Verbindungen besitzen wertvolle pharmakologische Eigenschaften, die sie gewerblich verwertbar machen. Als selektive Zyklisch-Nukleotid Phosphodiesterase (PDE) Inhibitoren (und zwar des Typs IV) eignen sie sich einerseits als Bronchialtherapeutika (zur Behandlung von Atemwegsobstruktionen aufgrund ihrer dilatierenden aber auch aufgrund ihrer atemfrequenz- bzw. atemantriebssteigernden Wirkung) und zur Behebung von erektiler Dysfunktion aufgrund der gefäßdilatierenden Wirkung, andererseits jedoch vor allem zur Behandlung von Erkrankungen, insbesondere entzündlicher Natur, z.B. der Atemwege (Asthma-Prophylaxe), der Haut, des Darms, der Augen und der Gelenke, die vermittelt werden durch Mediatoren, wie Histamin, PAF (Plättchen- aktivierender Faktor), Arachidonsäure-Abkömmlinge wie Leukotriene und Prostaglandine, Zytokine, Interleukine, Chemokine, alpha-, beta- und gamma-Interferon, Tumornekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS, Multiple Sklerose), Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise erektile Dysfunktion oder Koliken der Nieren und der Harnleiter im Zusammenhang mit Nierensteinen; oder auch Erkrankungen des ZNS, wie beispielsweise Depressionen oder arteriosklerotische Demenz.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,01 und 0,5 mg/kg. Die übliche Dosis bei systemischer Therapie liegt zwischen 0,05 und 2 mg pro Tag.

### Biologische Untersuchungen

Bei der Untersuchung der PDE IV-Hemmung auf zellulärer Ebene kommt der Aktivierung von Entzündungszellen besondere Bedeutung zu. Als Beispiel sei die FMLP (N-formyl-methionyl-leucyl-phenylalanin)-induzierte Superoxid-Produktion von neutrophilen Granulozyten genannt, die als Luminol-verstärkte Chemolumineszenz gemessen werden kann. [Mc Phail LC, Strum SL, Leone PA und Sozzani S, The neutrophil respiratory burst mechanism. In "Immunology Series" **1992,** 57: 47-76; ed. Coffey RG (Marcel Decker, Inc., New York-Basel -Hong Kong)].

Substanzen, welche die Chemolumineszenz sowie die Zytokinsekretion und die Sekretion entzündungssteigernder Mediatoren an Entzündungszellen, insbesonders neutrophilen und eosinophilen Granulozyten hemmen, sind solche, welche die PDE IV hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE IV-Hemmung durch die erfindungsgemäßen Substanzen ist damit ein zentraler Indikator für die Unterdrückung von entzündlichen Prozessen. (**Giembycz MA,** Could isoenzyme-selective phosphodiesterase inhibitors render bronchodilatory therapy redundant in the treatment of bronchial asthma?. Biochem Pharmacol **1992,** 43, 2041-2051; **Torphy TJ** et al., Phosphodiesterase inhibitors: new opportunities for treatment of asthma. Thorax **1991,** 46, 512-523; **Schudt C** et al., Zardaverine: a cyclic AMP PDE III/IV inhibitor. In "New Drugs for Asthma Therapy", 379-402, Birkhäuser Verlag Basel **1991**; **Schudt C** et al., Influence of selective phosphodiesterase inhibitors on human neutrophil functions and levels of cAMP and Caᵢ. Naunyn-Schmiedebergs Arch Pharmacol **1991,** 344, 682-690; **Nielson CP** et al., Effects of selective phosphodiesterase inhibitors on polymorphonuclear leukocyte respiratory burst. J Allergy Clin Immunol **1990**, 86, 801-808; Schade et al., The specific type III and IV phosphodiesterase inhibitor zardaverine suppress formation of tumor necrosis factor by macrophages. European Journal of Pharmacology **1993,** 230, 9-14).

### 1. Hemmung der PDE IV-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. **1980,** 311, 193-198). Hierbei erfolgt im ersten Schritt die PDE-Reaktion. In einem zweiten Schritt wird das entstandene 5'-Nukleotid durch eine 5'-Nukleotidase des Schlangengiftes von ophiophagus hannah (King Cobra) zum ungeladenen Nukleosid gespalten. Im dritten Schritt wird das Nukleosid auf Ionenaustauschsäulen vom verbliebenen geladenen Substrat getrennt. Die Säulen werden mit 2 ml 30 mM Ammoniumformiat (pH 6,0) direkt in Minivials eluiert, in die noch 2 ml Szintillatorflüssigkeit zur Zählung gegeben wird.

Die für die erfindungsgemäßen Verbindungen ermittelten Hemmwerte ergeben sich aus der folgenden Tabelle A, in der die Nummern der Verbindungen den Nummern der Beispiele entsprechen.

**Tabelle A**

| Hemmung der PDE IV-Aktivität | |
|---|---|
| Verbindung | PDE 4 -logIC50 |
| 1 | 5,88 |
| 2 | 7,10 |
| 3 | 7,59 |
| 4 | 6,12 |
| 5 | 6,73 |
| 6 | 6,36 |
| 7 | 5,95 |
| 8 | 6,95 |
| 9 | 7,01 |
| 10 | 6,76 |

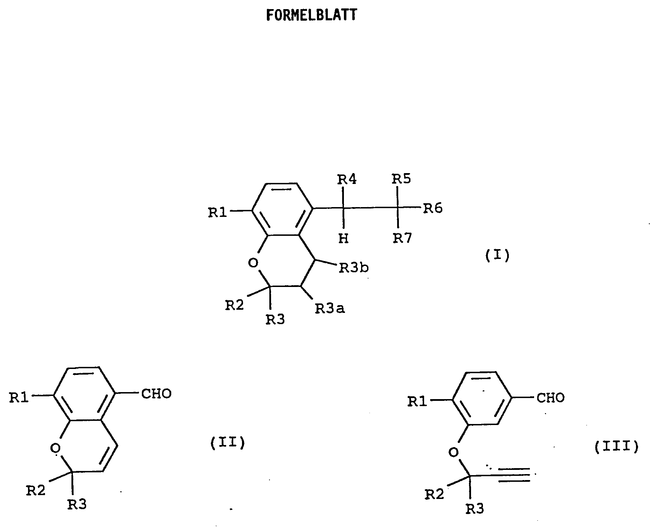

## Patentansprüche

1. Verbindungen der Formel I, 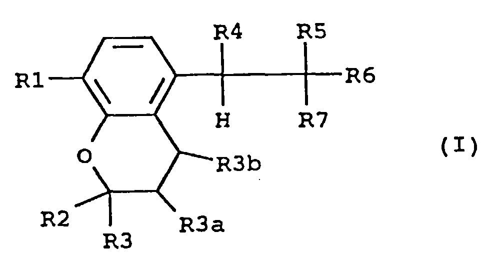 worin
R1 1-4C-Alkoxy oder ganz oder überwiegend durch Fluor substituiertes 1-4C-Alkoxy bedeutet,
R2 Wasserstoff, 1-7C-Alkyl, 3-7C-Cycloalkyl oder 3-7C-Cycloalkylmethyl bedeutet,
R3 Wasserstoff, 1-7C-Alkyl, 3-7C-Cycloalkyl oder 3-7C-Cycloalkylmethyl bedeutet,
oder worin
R2 und R3 gemeinsam und unter Einschluß des Kohlenstoffatoms, an das beide gebunden sind, einen 3-7C-Cycloalkylrest darstellen,
R3a und R3b jeweils Wasserstoff oder gemeinsam eine weitere C-C-Bindung bedeuten,
R4 Wasserstoff oder Hydroxy bedeutet,
R5 Wasserstoff bedeutet,
oder worin
R4 und R5 gemeinsam eine weitere C-C-Bindung darstellen,
R6 Wasserstoff oder Cyano bedeutet,
R7 einen Pyridylrest bedeutet,
die Salze dieser Verbindungen sowie die N-Oxide der Pyridine und deren Salze.

2. Verbindungen der Formel I, nach Anspruch 1, worin
R1 1-2C-Alkoxy bedeutet,
R2 Wasserstoff, 1-4C-Alkyl oder 3-5C-Cycloalkyl bedeutet,
R3 Wasserstoff, 1-4C-Alkyl oder 3-5C-Cycloalkyl bedeutet,
oder worin
R2 und R3 gemeinsam und unter Einschluß des Kohlenstoffatoms, an das beide gebunden sind, einen 3-7C-Cycloalkylrest darstellen,
R3a und R3b jeweils Wasserstoff oder gemeinsam eine weitere C-C-Bindung bedeuten,
R4 Wasserstoff oder Hydroxy bedeutet,
R5 Wasserstoff bedeutet,
oder worin
R4 und R5 gemeinsam eine weitere C-C-Bindung darstellen,
R6 Wasserstoff oder Cyano bedeutet,
R7 einen Pyridylrest bedeutet,
sowie die Salze dieser Verbindungen.

3. Verbindungen der Formel I, nach Anspruch 1, worin
R1 1-2C-Alkoxy bedeutet,
R2 1-4C-Alkyl oder 3-5C-Cycloalkyl bedeutet,
R3 Wasserstoff oder 1-4C-Alkyl bedeutet,
oder worin
R2 und R3 gemeinsam und unter Einschluß des Kohlenstoffatoms, an das beide gebunden sind, einen Cyclopropyl-, Cyclobutyl- oder Cyclopentylring darstellen, und
R3a und R3b jeweils Wasserstoff oder gemeinsam eine weitere C-C-Bindung bedeuten,
R4 Wasserstoff oder Hydroxy bedeutet,
R5 Wasserstoff bedeutet,
oder worin
R4 und R5 gemeinsam eine weitere C-C-Bindung darstellen,
R6 Wasserstoff oder Cyano bedeutet,
R7 einen Pyridylrest bedeutet,
sowie die Salze dieser Verbindungen.

4. Verbindungen der Formel I, nach Anspruch 1, worin
R1 Methoxy bedeutet,
R2 1-4C-Alkyl bedeutet und
R3 1-4C-Alkyl bedeutet,
oder worin
R2 und R3 gemeinsam und unter Einschluß des Kohlenstoffatoms, an das beide gebunden sind, einen Cyclopropyl-, Cyclobutyl- oder Cyclopentylring darstellen,
R3a und R3b jeweils Wasserstoff oder gemeinsam eine weitere C-C-Bindung bedeuten,
R4 Wasserstoff bedeutet,
R5 Wasserstoff bedeutet,
oder worin
R4 und R5 gemeinsam eine weitere C-C-Bindung darstellen,
R6 Wasserstoff oder Cyano bedeutet,
R7 einen Pyridylrest bedeutet,
oder
R1 Methoxy bedeutet,
R2 1-4C-Alkyl bedeutet und
R3 1-4C-Alkyl bedeutet,
oder worin
R2 und R3 gemeinsam und unter Einschluß des Kohlenstoffatoms, an das beide gebunden sind, einen Cyclopropyl-, Cyclobutyl- oder Cyclopentylring darstellen,
R3a und R3b jeweils Wasserstoff oder gemeinsam eine weitere C-C-Bindung bedeuten,
R4 Hydroxy bedeutet,
R5 Wasserstoff bedeutet,
R6 Wasserstoff bedeutet,
R7 einen Pyridylrest bedeutet,
sowie die Salze dieser Verbindungen.

5. Verfahren zur Herstellung der Verbindungen der Formel I nach Anspruch 1 und ihrer Salze, sowie der N-Oxide der Pyridine und deren Salze, dadurch gekennzeichnet, daß man
a) zur Herstellung von Verbindungen der Formel I, worin R1, R2 und R3 die in Anspruch 1 genannten Bedeutungen besitzen, R3a und R3b gemeinsam eine C-C-Bindung bedeuten, R4 Hydroxy bedeutet, R5 und R6 Wasserstoff bedeuten und R7 die in Anspruch 1 genannten Bedeutungen besitzt, entsprechende Verbindungen der Formel II, 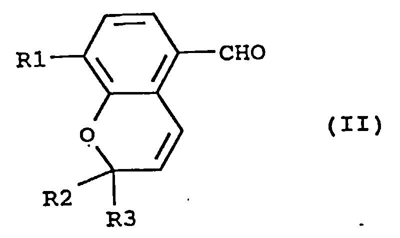 worin R1, R2 und R3 die in Anspruch 1 genannten Bedeutungen besitzen mit entsprechenden Verbindungen der Formel M-C(R5)(R6)(R7), worin R5 und R6 Wasserstoff bedeuten und R7 die in Anspruch 1 genannten Bedeutungen besitzt und M ein geeignetes Metallatom darstellt, umsetzt oder daß man
b) zur Herstellung von Verbindungen der Formel I, worin R1, R2, R3, R6 und R7 die in Anspruch 1 genannten Bedeutungen besitzen und worin R3a, R3b, R4 und R5 Wasserstoff bedeuten entsprechende Verbindungen der Formel I, worin R1, R2, R3, R6 und R7 die in Anspruch 1 genannten Bedeutungen besitzen und worin R3a gemeinsam mit R3b und/oder R4 gemeinsam mit R5 eine weitere C-C-Bindung bedeutet hydriert, oder daß man
c) zur Herstellung von Verbindungen der Formel I, worin R1, R2, R3, R3a, R3b, R6 und R7 die in Anspruch 1 genannten Bedeutungen besitzen und R4 und R5 gemeinsam eine C-C-Bindung bedeuten entsprechende Verbindungen der Formel I, worin R1, R2, R3, R3a, R3b, R6 und R7 die in Anspruch 1 genannten Bedeutungen besitzen, R4 Hydroxy und R5 Wasserstoff bedeutet, dehydratisiert, oder daß man
d) zur Herstellung von Verbindungen der Formel I, worin R1, R2, R3 und R7 die in Anspruch 1 genannten Bedeutungen besitzen, R3a und R3b sowie R4 und R5 jeweils gemeinsam eine C-C-Bindung bedeuten und R6 Cyano bedeutet, entsprechende Verbindungen der Formel II, worin R1, R2 und R3 die in Anspruch 1 genannten Bedeutungen haben mit entsprechenden Verbindungen der Formel NC-CH₂-R7, worin R7 die in Anspruch 1 genannten Bedeutungen hat, in Gegenwart einer Base umsetzt,
und daß man gewünschtenfalls anschließend nach a), b), c) oder d) erhaltene Verbindungen der Formel I in ihre Salze oder in die N-Oxide der Pyridine und gewünschtenfalls anschließend in die Salze überführt, oder daß man gewünschtenfalls anschließend erhaltene Salze der Verbindungen der Formel I in die freien Verbindungen überführt.

6. Arzneimittel enthaltend eine oder mehrere Verbindungen nach Anspruch 1 zusammen mit üblichen pharmazeutischen Hilfs- und/oder Trägerstoffen.

7. Verbindungen nach Anspruch 1 zur Anwendung bei der Behandlung von Krankheiten.

8. Verwendung von Verbindungen nach Anspruch 1 zur Herstellung von Arzneimitteln für die Behandlung von Atemwegserkrankungen.

9. Verwendung von Verbindungen nach Anspruch 1 zur Herstellung von Arzneimitteln für die Behandlung von Dermatosen.
